# EUROPEAN PATENT APPLICATION

(11) **EP 4 454 482 A1**
(43) Date of publication of application: **30.10.2024**
(21) Application number: 23169992.7
(22) Date of filing: 26.04.2023
(51) Int. Cl.: A23L 27/40, A23L 27/00, A61K 31/7028

(54) **PREPARATIONS COMPRISING OLEANOL GLYCOSIDES**

(71) Applicant: Analyticon Discovery GmbH, 14473 Potsdam (DE)
(72) Inventor: SIEMS, Karsten, 14552 Michendorf (DE); THIER, Isabel, 14482 Potsdam (DE)
(74) Representative: Fabry, Bernd

(57) **Abstract**

Suggested is a preparation, comprising or consisting of
(a) specific oleanol glycosides, their salts or an extract containing oleanol glycosides and
(b) inorganic salts or mineral substances.

## Description

### AREA OF INVENTION

The present invention refers to the area of food and pharmaceutical products and relates to new preparations containing escin or compounds extracted from escin for taste modulation and corresponding methods and uses of these substances.

### BACKGROUND OF THE INVENTION

The purchasing and consuming behaviour of consumers has been changing significantly. Overconsumption of sodium, contained as sodium chloride in many food products, is held responsible for many cardiovascular health issues within the modern society. In addition to the reduction of sugar it is therefore desirable to reduce the intake of sodium via foodstuffs. One possibility is the replacement of sodium (especially table salt or sodium chloride) by potassium salts (esp. potassium chloride). Potassium chloride exhibits apart from the salty taste an unpleasant off-taste, which is frequently described as metallic, bitter, chemical and acrid. Another possibility is the enhancement of salty taste of sodium by salt taste enhancers to reach the same salty taste in a preparation by less sodium.

For this reason, there is an intensive demand in the market for products, which are increasing the pleasantness of sodium chloride replacers. This includes products, which increase the saltiness or are capable of masking, suppressing or modulating the unpleasant taste impressions of potassium salts, e.g., potassium chloride as well as other chloride salts such as magnesium chlorides, and calcium chlorides, i.e., providing them with a pleasant taste. The scientific literature with a focus on patents about potassium-based salt substitutes was reviewed by J. Ranilovic (Comprehensive Reviews in Food Science and Food Safety, 2017, Vol 18, p. 881 - 894). The latter publication states that the room for improvement in the area of taste improving agents is still very large.

Potassium salts are added to foods, but usually not with the intention to balance a potassium deficiency, but as a substitute for salt in order to reduce the sodium content in foods while maintaining the taste of salt. As potassium chloride has an unpleasant side taste, only part of the sodium may be substituted by potassium. In order to reduce the unpleasant taste of potassium chloride, for example, ascorbic acid, fumaric acid and citric acid, or various sugars (lactose, dextrose), or yeast extract may be added (cf. DE 3035518 C2). However, these solutions are only suitable for some foods, as they are not neutral with regard to their taste or may lead to an increased uptake of calories, which is not desired in sodium-chloride reduced foods, which are, therefore, healthier.

Magnesium is an important mineral substance and is added to foods as a taste molecule as well as a technological additive to reduce sodium content. Another application is under-supply with magnesium which is often not diagnosed, but it may appear as a side effect in many diseases (e.g., diabetes, hypertension). Senior citizens and pregnant women frequently have an increased demand for magnesium. An increased demand for magnesium may be balanced by mineral supplements or by adding magnesium to foods, e.g., bread that is specifically produced for senior citizens. Magnesium is added to food in the form of various salts, e.g., as magnesium chloride, magnesium sulfate, phosphates, carbonates or as carnalite, kainite and schoenite or as glutamate (JP 2010 011807 A). Many magnesium-containing salts taste bitter, particularly magnesium sulfate, which is also referred to as epsomite.

Calcium is an important mineral substance, too, and is added to foods as a taste molecule as well as a technological additive to reduce sodium content. Calcium is added in the form of various salts, e.g., as chlorides, sulphates, phosphates, or carbonates. Calcium containing salts taste unpleasant, particularly calcium chloride.

Chloride is a counterion of important mineral salts, which is added to foods together with potassium, calcium, magnesium or ammonium as a salty taste molecule as well as a technological additive to reduce sodium content. Chloride salts of potassium, calcium and magnesium have an intrinsic saltiness, but also a strong unpleasant side taste.

### RELEVANT PRIOR ART

Taste-modulating natural substances, including, specifically, the so-called "bitter blockers" are already known from the state of the art.

DE 10 2012 214560 A1 (SYMRISE) discloses the use of enterodiol compounds comprising (2R,3S)-2,3-bis-(3-hydroxy-benzyl)-butane-1,4-diol, (2S,3R)-2,3-bis-(3-hydroxybenzyl)-butane-1,4-diol and (2R,3R)-2,3-bis-(3-hydroxy-benzyl)-butane-1,4-diol and/or salts for sensorially changing or masking unpleasant taste impression, preferably bitter-, astringent- and/or metallic taste impression of an unpleasant tasting substance, preferably a bitter substance.

EP 1258200 A2 (SYMRISE) relates to hydroxy flavanones, their salts and stereoisomers and their mixtures for suppressing or reducing the bitter and/or metallic taste impression. In addition, the document relates to nutritional, nutritive or consumable preparations or oral pharmaceutical compositions, according to which the compounds have an active content of hydroxyflavanones, their salts and stereoisomers or their mixtures.

EP 2559346 A1 (SYMRISE) relates to the use of certain oleanene-type triterpene glycosides for modifying (i.e., modulating) or masking (i.e. reducing or suppressing) unpleasant taste impressions, in particular bitter and/or astringent taste impressions and to a corresponding method for modulating or masking an unpleasant taste of a substance or substance mixture.

WO 2011 050955 A1 (DEUTSCHES INST ERNAHRUNGSFORSCHUNG) relates to antagonists and agonists of the human bitter-taste receptors hTAS2R40, hTAS2R43, hTAS2R44, hTAS2R46, and hTAS2R47. The document also relates to methods for identifying further molecules that suppress or enhance bitter taste transduction or bitter taste response mediated by hTAS2R40, hTAS2R43, hTAS2R44, hTAS2R46, and/or hTAS2R47 and uses thereof.

WO 2013 072332 A1 (GIVAUDAN) relates to compositions and methods of using (-)-hardwickiic acid, in free or orally acceptable base addition salt form, to mask or decrease a subject's perception of bitter taste and/or enhance perception of sweet taste.

US 2015 0374021 A1 (GENERAL MILLS) discloses more than hundred naturally-derived compounds having rather different structures for improving the perception of salty taste, enhance the perception of salty taste of a salt, or act at one or more sodium channels.

### OBJECT OF THE INVENTION

Thus, the state of the art sufficiently describes the use of natural substances, specifically of phenol derivatives, to counteract, cover, or change undesired taste properties. However, the disadvantages of the compositions mentioned are that they require high quantities, and that they only have very specific effects, particularly with respect potassium chlorides. In the quantities described, these substances also exhibit a taste of their own which adulterates the desired taste experience, usually with a deteriorating effect. In addition, the effectiveness of these active agents strongly depends on the matrix in which they are used. For producers of foods, it is, therefore, of great importance to be able to select from a large number of alternative active agents in order to be able to determine the optimal substance for the particular purpose of application.

The object of the present invention was, therefore, to provide a natural substance, which is capable of covering, neutralising, or advantageously changing unpleasant taste impressions of inorganic salts e.g., potassium salts such as chlorides and sulfates as well as other chlorides such as magnesium chloride and calcium chloride, even when present in very small quantities.

### BRIEF DESCRIPTION OF THE INVENTION

A first object of the present invention refers to a preparation, comprising or consisting of
(a) at least one oleanol glycoside and/or its salt and/or an extract containing at least one oleanol glycoside and
(b) at least one inorganic salt or mineral substance,
   wherein compound (a) follows formula 1. 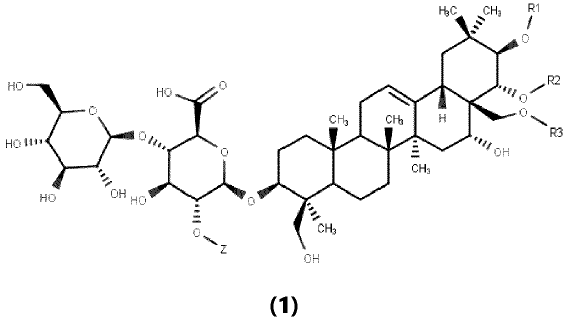
   wherein Z represents at least one monosaccharide selected from Glc, Gal, Xyl, Ara and Rha; R1 has the meaning of Ac, Ang, Tig, MeBu, and/or iBu, R2 and R3 independently stand for H or Ac,
   on condition that a compound (a) according to formula (I) in which Z means Glc, R1 means Tig, R2 is H, and R3 is H is disclaimed.

The invention provides a natural compound capable of improving the pleasantness of sodium chloride replacers and thereby enables further sodium reduction in food products, which was not possible before.

Surprisingly, it was found that oleanol glycosides obtainable or obtained from *Aesculus hippocastanum* (commonly known as escin or aescin) fully meet the complex profile of requirements described in the beginning. It improves the taste of inorganic alkali metal salts and alkaline earth metal salts having unpleasant off-flavours like metallic, bitter, acrid or chemical, especially inorganic potassium salts as well as chloride salts. It is a purely plant-based product which is fully effective also in very small concentrations of, for example, 10 µM (ca. 11 mg/l), and which thus proves to be superior to alternative prior-art substances.

Taste impressions, particularly unpleasant taste impressions that are intended to be improved within the meaning of the invention are understood as metallic, bitter, chemical and acrid taste sensations. Moreover, pleasant taste impressions, e.g., saltiness or spiciness and flavours are intended to be improved within the meaning of the invention. The overall improvement of unpleasant and pleasant tastes leads to a superior taste impression.

### Oleanol glycosides from Aesculus sp.

Plant species of the genus Aesculus contain glycosides of the aglycon 12-Oleanene-3,16,21,22,24,28-hexol. A sugar side chain consisting of three monosaccharides (one of them is glucuronic acid) is attached at O-3. O-23 is esterified with tiglic acid, angelic acid, i-butyric acid, 2-methyl-butyric acid or acetic acid, respectively. O-24 and/or O-28 are esterified with acetic acid.

Particularly preferred are compounds of formula (1) selected from the following list:

| **Structure** | **Name / CAS** |
|---|---|
| 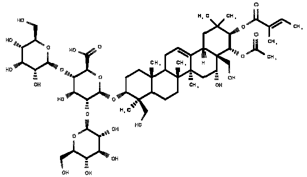 | Escin Ia |
| 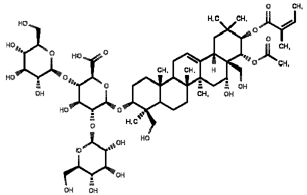 | Escin Ib |
| 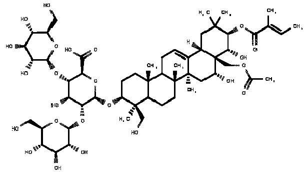 | Escin IVa, Isoescin Ia |
| 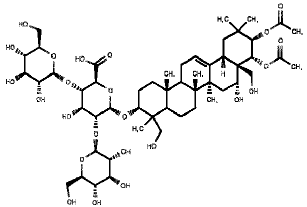 | Escin IV |
| | 219944-22-6 |
| 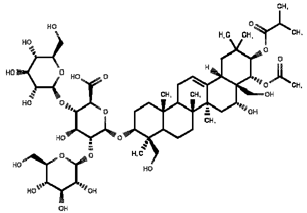 | Escin V |
| | [219944-26-0] |

More preferably the oleanol glycosides are selected from the group comprising Escin la, Escin Ib, Escin Ila, Escin Ilb, Escin Illa, Escin Illb, Escin IVa, Escin IVb, Escin IV, Escin V, Escin VI, or a mixture thereof. More preferably the oleanol glycosides are selected from the group comprising Escin la, Escin Ib, Escin Ila, Escin Ilb, Escin Illa, Escin Illb, or a mixture containing these compounds. The most preferable oleanol glycosides are selected from the group comprising Escin la or Escin Ib, or a mixture containing these compounds. Instead of using the actual oleanol glycosides itself it is also possible to use its salts, specifically its alkali metals salts, alkaline earth metals salts or ammonium salts.

According to the present application extracts containing oleanol glycosides can also be used, the preparation of which is known to the skilled person in the art and described by way of example, in example 1 below. Preferably, these extracts contain at least 10% (w/v), more preferably at least 30 to 99 % (w/v) and particularly preferred about 50 to 99 % (w/v) Oleanol glycosides.

For the preparation of extracts containing oleanol glycosides different plant sources can be used. Preferably the extracts are obtained from the genus Aesculus, more preferably from the *Aesculus hippocastanum* (including hybrids), *A. chinensis, A. pavia,* and most preferably from seeds *Aesculus hippocastanum.*

The conventional different extraction methods can be used for the preparation of Oleanol glycosides containing extracts. Preferably the solvents are selected from the group comprising water, C1 to C4-alcohols, acetone, methyl ethyl ketone, preferably selected from the group comprising water, methanol, ethanol, and acetone. The solvents can be used alone or in a mixture.

### Inorganic salts and mineral substances

According to the present application the inorganic salts or mineral substances forming component (b) are any substances serving as sodium chloride replacers in food products, such as inorganic alkali metal salts as well as alkaline earth metal salts, for example potassium, magnesium and calcium salts and their mixtures.

Preferably, the inorganic salts or mineral substances are selected from the group consisting of sodium, potassium, magnesium, and calcium and the anions are selected from the group consisting of chloride, sulfate, phosphate, carbonate, and glutamate. Even more preferred are preparations wherein the inorganic salts or mineral substances are selected from the group comprising potassium chloride, potassium sulfate, chlorides, sulfates, phosphates, carbonates of magnesium and calcium. Moist preferred are sodium chloride, potassium chloride or their mixtures. Minerals such a karnalite, kainite, schoenite and ammonium chloride also belong to the inorganic salts or mineral substances according to the present application.

### Preparations

According to the present invention said preparations may comprise component (a) in concentration of from about 0.01 to about 500 ppm, preferably from about 0.05 to about 100 ppm and more preferably from about 0.1 to about 50 ppm. The preparation may comprise component (b) in concentration of from about 0.01 wt.-percent to about 5 wt.-percent, and preferably from about 0.05 to about 2 wt.-percent. More particularly, the preparations according to the present invention may comprises component (a) and (b) in the mass ratio of from 1:50 to 1:5000.

In a particularly preferred embodiment said preparations may comprise component (a) in concentration of from about 0.01 to about 500 ppm and component (b) in concentration of from about 0.01 wt.-percent to about 5 wt.-percent.

A form of preparation which is intended for commercial distribution and which is particularly suitable, for example, in form of a table salt substitute mixture, which contains 20 to 60 % potassium chloride, 30 to 70 % sodium chloride, about 10 % other mineral salts like calcium or magnesium salts as well as other components like.

A further form of preparation which is intended for commercial distribution and which is particularly suitable, for example, in form of a seasoning flavour packages for food products. Such flavour packages contain mineral salts, e.g. sodium chloride; sodium nitrate; sodium sulphate; sodium citrate; sodium-L-lactate; sodium gluconate; sodium glutamate; ammonium chloride; potassium chloride; potassium glutamate; potassium citrate; potassium sulphate; potassium citrates; potassium-L-lactate; potassium formate; potassium succinate; potassium fumarate; carnalite; kainite; schoenite; magnesium chloride; magnesium sulphate; magnesium carbonate; magnesium citrate; magnesium formate; magnesium gluconate; calcium chloride; calcium sulphate; calcium phosphate; calcium carbonate; calcium citrate; calcium formate; calcium gluconate; calcium glutamate; calcium glycerophosphate; essential micro-minerals such as iron, manganese, copper, zinc and cobalt; seasoning herbs; herbal extracts; vegetables; spices; anticaking agents like silicon dioxide, tricalcium phosphate; carbohydrates, like sucrose, glucose, lactose, fructose, glycerol; sugar alcohols, such as sorbitol, maltitol, lac-titol, mannitol, xylitol; sugar substitutes; glycyrrhizin; sodium glycyrrhizinate; high intensity sweeteners, like sucralose, saccharin, aspartame, advantame, acesulfame K, cyclamate; yeast products, such as yeast extracts, autolysed yeast, hydrolysed yeast; hydrolysed animal and vegetable proteins; choline chloride; amino acids such as L-lysine, L-histidine, L-ornithine; citric acid; malic acid; tartaric acid; fumaric acid; lactic acid; acetic acid; benzoic acid; adipic acid; gluconic acid; food polymers such as maltodextrin, dextrin, starch, polydextrose, inulin, gum arabic, guar gum, xanthan gum, Karaya gum, carrageenan, agar-agar, pectin, rice flour, sodium alginate, polyethylene glycol, sodium carboxymethyl cellulose, methyl cellulose, proteins, gluten; folic acid; ascorbic acid; glutamic acid; monopotassium L-glutamate; monosodium L-glutamate, monoammonium L-glutamate; calcium di-L-glutamate; magnesium di-L-glutamate; guanylic acid; disodium guanylate; dipotassium guanylate; calcium guanylate; inosinic acid; disodium inosinate; dipotassium inosinate; calcium inosinate; calcium 5'-ribonucleotides; disodium 5'-ribonucleotides, taurine, betaine.

### FOOD PRODUCTS

Another object of the present invention is a food product comprising the preparation as explained above.

The food composition may be any composition which is suitable for consumption and are used for nutrition or enjoyment purposes, and are generally products which are intended to be introduced into the human or animal oral cavity, to remain there for a certain time and then either be eaten (e.g. ready-to-eat foodstuffs or feeds, see also herein below) or removed from the oral cavity again (e.g. chewing gums). Such products include any substances or products which in the processed, partially processed or unprocessed state are to be ingested by humans or animals. They also include substances which are added to orally consumable products during their manufacture, preparation or treatment and which are intended to be introduced into the human or animal oral cavity.

Food compositions according to the present invention in particular encompass chewing gums, candies, soft drinks, non- alcoholic and alcoholic beverages and in particular bakery products, convenience products (snacks) and soups.

Preferably, the food compositions comprise the preparations as defined above in a working amount, for example about 0.1 to about 10 % b.w., preferably about 0.5 to about 8 % b.w. and particularly from about 1 to about 5 % b.w. - calculated on the composition(s).

The food compositions according to the invention also include substances which in the unchanged, treated or prepared state are to be swallowed by a human or animal and then digested; in this respect, the orally consumable products according to the invention also include casings, coatings or other encapsulations which are to be swallowed at the same time or which may be expected to be swallowed. The expression "orally consumable product" covers ready-to-eat foodstuffs and feeds, that is to say foodstuffs or feeds that are already complete in terms of the substances that are important for the taste. The expressions "ready-to-eat foodstuff" and "ready-to-eat feed" also include drinks as well as solid or semi-solid ready-to-eat foodstuffs or feeds. Examples which may be mentioned are frozen products, which must be thawed and heated to eating temperature before they are eaten. Products such as yoghurt or ice-cream as well as chewing gums or hard caramels are also included among the ready-to-eat foodstuffs or feeds.

Preferred food compositions according to the invention also include "semi-finished products". Within the context of the present text, a semi-finished product is to be understood as being an orally consumable product which, because of a very high content of flavourings and taste-imparting substances, is unsuitable for use as a ready-to-eat orally consumable product (in particular foodstuff or feed). Only by mixing with at least one further constituent (e.g. by reducing the concentration of the flavourings and taste-imparting substances in question) and optionally further process steps (e.g. heating, freezing) is the semi-finished product converted into a ready-to-eat orally consumable product (in particular foodstuff or feed). Examples of semi-finished products which may be mentioned here are

Food composition according to the invention preferably comprises one or more preparations for nutrition or enjoyment purposes. These include in particular (reduced-calorie) baked goods (e.g. bread, dry biscuits, cakes, other baked articles), confectionery (e.g. chocolates, chocolate bars, other products in bar form, fruit gums, dragées, hard and soft caramels, chewing gum), non-alcoholic drinks (e.g. cocoa, coffee, green tea, black tea, (green, black) tea drinks enriched with (green, black) tea extracts, rooibos tea, other herbal teas, fruit-containing soft drinks, isotonic drinks, refreshing drinks, nectars, fruit and vegetable juices, fruit or vegetable juice preparations), instant drinks (e.g. instant cocoa drinks, instant tea drinks, instant coffee drinks), meat products (e.g. ham, fresh sausage or raw sausage preparations, spiced or marinated fresh or salt meat products), eggs or egg products (dried egg, egg white, egg yolk), cereal products (e.g. breakfast cereals, muesli bars, precooked ready-to-eat rice products), dairy products (e.g. full-fat or reduced-fat or fat-free milk drinks, rice pudding, yoghurt, kefir, cream cheese, soft cheese, hard cheese, dried milk powder, whey, butter, buttermilk, partially or completely hydrolysed milk-protein-containing products), products made from soy protein or other soybean fractions (e.g. soy milk and products produced therefrom, drinks containing isolated or enzymatically treated soy protein, drinks containing soy flour, preparations containing soy lecithin, fermented products such as tofu or tempeh or products produced therefrom and mixtures with fruit preparations and optionally flavours), fruit preparations (e.g. jams, sorbets, fruit sauces, fruit fillings), vegetable preparations (e.g. ketchup, sauces, dried vegetables, frozen vegetables, precooked vegetables, boiled-down vegetables), snacks (e.g. baked or fried potato crisps or potato dough products, maize- or groundnut-based extrudates), fat- and oil-based products or emulsions thereof (e.g. mayonnaise, re-moulade, dressings, in each case full-fat or reduced-fat), other ready-made dishes and soups (e.g. dried soups, instant soups, precooked soups), spices, spice mixtures and in particular seasonings which are used, for example, in the snacks field, sweetener preparations, tablets or sachets, other preparations for sweetening or whitening drinks or other foods. The preparations within the scope of the invention can also be used in the form of semi-finished products for the production of further preparations for nutrition or enjoyment purposes. The preparations within the scope of the invention can also be in the form of capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes, or in the form of other preparations which can be swallowed or chewed, and in the form of food supplements.

The preparations can also be in the form of capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes, or in the form of other preparations which can be swallowed or chewed, for example in the form of food supplements.

The semi-finished products are generally used for the production of ready-to-use or ready-to-eat preparations for nutrition or enjoyment purposes.

Further constituents of a ready-to-eat preparation or semi-finished product for nutrition or enjoyment purposes can be conventional base substances, auxiliary substances and additives for foods or enjoyment foods, for example water, mixtures of fresh or processed, vegetable or animal base or raw substances (e.g. raw, roast, dried, fermented, smoked and/or boiled meat, bone, cartilage, fish, vegetables, herbs, nuts, vegetable juices, vegetable pastes or mixtures thereof), digestible or non-digestible carbohydrates (e.g. sucrose, maltose, fructose, glucose, dextrins, amylose, amylopectin, inulin, xylans, cellulose, tagatose), sugar alcohols (e.g. sorbitol, erythritol), natural or hardened fats (e.g. tallow, lard, palm fat, cocoa fat, hardened vegetable fat), oils (e.g. sunflower oil, groundnut oil, maize germ oil, olive oil, fish oil, soya oil, sesame oil), fatty acids or their salts (e.g. potassium stearate), proteinogenic or non-proteinogenic amino acids and related compounds (e.g. γ-aminobutyric acid, taurine), peptides (e.g. glutathione), natural or processed proteins (e.g. gelatin), enzymes (e.g. pepti-dases), nucleic acids, nucleotides, taste correctors for unpleasant taste impressions, further taste modulators for further, generally not unpleasant taste impressions, other taste-modulating substances (e.g. inositol phosphate, nucleotides such as guanosine monophosphate, adenosine monophosphate or other substances such as sodium glutamate or 2-phenoxypropionic acid), emulsifiers (e.g. lecithins, diacylglycerols, gum arabic), stabilisers (e.g. carrageenan, alginate), preservatives (e.g. benzoic acid and its salts, sorbic acid and its salts), antioxidants (e.g. tocopherol, ascorbic acid), chelators (e.g. citric acid), organic or inorganic acidifying agents (e.g. acetic acid, phosphoric acid), additional bitter substances (e.g. quinine, caffeine, limonene, amarogentine, humulone, lupulone, catechols, tannins), substances that prevent enzymatic browning (e.g. sulfite, ascorbic acid), ethereal oils, plant extracts, natural or synthetic colourings or colouring pigments (e.g. carotinoids, flavonoids, anthocyans, chlorophyll and derivatives thereof), spices, trigeminally active substances or plant extracts containing such trigeminally active substances, synthetic, natural or nature-identical flavourings or odorants as well as odour correctors.

Food compositions according to the invention, for example those in the form of preparations or semi-finished products, preferably comprise a flavour composition in order to complete and refine the taste and/or odour. A preparation can comprise as constituents a solid carrier and a flavour composition. Suitable flavour compositions comprise, for example, synthetic, natural or nature-identical flavourings, odorants and taste-imparting substances, reaction flavourings, smoke flavourings or other flavour-giving preparations (e.g. protein (partial) hydrolysates, preferably protein (partial) hydrolysates having a high arginine content, barbecue flavourings, plant extracts, spices, spice preparations, vegetables and/or vegetable preparations) as well as suitable auxiliary substances and carriers. Particularly suitable here are the flavour compositions or constituents thereof which produce a roasted, meaty (in particular chicken, fish, seafood, beef, pork, lamb, mutton, goat), vegetable-like (in particular tomato, onion, garlic, celery, leek, mushroom, aubergine, seaweed), spicy (in particular black and white pepper, cardamom, nutmeg, pimento, mustard and mustard products), fried, yeast-like, boiled, fatty, salty and/or pungent flavour impression and accordingly can enhance the spicy impression. The flavour compositions generally comprise more than one of the mentioned ingredients.

The food compositions of the present invention are preferably selected from the group comprising
- baked goods (e.g. bread, dry biscuits, cakes, other baked articles)
- confectionery, preferably reduced-calorie or calorie-free confectionery, preferably selected from the group comprising muesli bar products, fruit gums, dragées, hard caramels and chewing gum,
- non-alcoholic drinks, preferably selected from the group comprising green tea, black tea, (green, black) tea drinks enriched with (green, black) tea extracts, rooibos tea, other herbal teas, fruit-containing low-sugar or sugar-free soft drinks, isotonic drinks, nectars, fruit and vegetable juices, fruit and vegetable juice preparations,
- instant drinks, preferably selected from the group comprising instant (green, black, rooibos, herbal) tea drinks,
- cereal products, preferably selected from the group comprising low-sugar and sugar-free breakfast cereals and muesli bars,
- dairy products, preferably selected from the group comprising reduced-fat and fat-free milk drinks, yoghurt, kefir, whey, buttermilk and ice-cream,
- products made from soy protein or other soybean fractions, preferably selected from the group comprising soy milk, products produced from soy milk, drinks containing isolated or enzymatically treated soy protein, drinks containing soy flour, preparations containing soy lecithin, products produced from preparations containing soy lecithin and mixtures with fruit preparations and optionally flavors,
- sweetener preparations, tablets and sachets,
- sugar-free dragées,
- ice-cream, with or without milk-based constituents, preferably sugar-free
- other ready-made dishes and soups (e.g. dried soups, instant soups, precooked soups).

### Food grade emulsifiers

The preferred food grade emulsifiers encompass the following groups:
- Lecithin;
- Fatty acid derivatives, such as for example polyglycerol esters (PGE), polysorbates ("TWEEN"), stearoyl lactylates, propylene glycol esters (PGMS), and sucrose esters;
- Polyglycerol Polyricinoleate (PGPR);
- Ammonium Phosphatide (AMP);
- Mono and Diglycerides of C₆-C₂₂ saturated or unsaturated fatty acids;
- and mixtures thereof.

### Aroma or flavoring compounds

Aroma compounds and flavoring agents forming group (c) are well known in the art and can be chosen from synthetic flavoring liquid and/or oils derived from plants leaves, flowers, fruits and so forth, and combinations thereof. Representative flavoring liquids include: artificial, natural or synthetic fruit flavors such as eucalyptus, lemon, orange, banana, grape, lime, apricot and grapefruit oils and fruit essences including apple, strawberry, cherry, orange, pineapple and so forth; bean and nut derived flavors such as coffee, cocoa, cola, peanut, almond and so forth; and root derived flavors such as licorice or ginger.

Suitable aroma or flavoring compounds encompass the following species:
- 12-methyltridecanal;
- acetaldehyde;
- acetanisole;
- acethylthiazole-2;
- acethylthiazoline-2;
- acetic acid;
- acetyl methylcarbinol;
- acetyl pyrazine-2;
- acetyl pyridine-2;
- allyl capronate;
- allyl isothiocyanate;
- amyl cinnamaldehyde-alpha;
- anethole;
- anisaldehyde-para;
- anisic alcohol;
- benzaldehyde;
- benzyl acetate;
- borneol-l;
- butyl acetate;
- butylidene phthalide-3;
- butyric acid;
- camphor;
- caproic acid;
- carvacrol;
- carvomenthone;
- carvon-d;
- carvon-l;
- carvylacetate-cis;
- caryophyllene;
- cineole-1;4;
- cineole-1;8;
- cinnamalcohol;
- cinnamaldehyde;
- cinnamyl acetate;
- citral;
- citronellal;
- citronellol;
- citronellyl acetate;
- cresol
- cuminaldehyde;
- cyclopentadecanolide;
- damascenone-alpha;
- damascenone-beta;
- damascone-alpha;
- damascoe-beta;
- decadienal-2;4;
- decadienal-2;4;
- decalactone delta;
- decalacton gamma;
- decanal;
- dehydromenthofurolactone;
- diallyl disulfide;
- diethylpyrazine-2;3;
- dihydroanethol;
- dihydrocarvone;
- dihydrocoumarin;
- dihydroionone-beta;
- dihydromenthofurolactone;
- dihydronootkatone;
- dimethyl lanthranilate;
- dimethyl isopropyldithiazine;
- dimethyl pyrazine;
- dimethyl sulfide;
- diphenyloxide;
- divanilline;
- d-menthol;
- d-menthone;
- d-menthyl acetate;
- dodecalactone-delta;
- dodecalactone-gamma;
- epithiomenthan-1;8;
- ethyl acetate;
- ethyl butyrate;
- ethyl capronate;
- ethyl caprylate;
- ethyl cinnamate;
- ethyl isobutyrate;
- ethyl isovaleria-nate;
- ethyl lactate;
- ethyl maltol;
- ethyl methyl butyrate-2;
- ethyl methyl thiopropionate;
- ethyl phenol-4;
- ethyl vanillin;
- eugenol;
- fenchol;
- filbertone;
- frambinone methyl ether;
- frambinone;
- furaneol;
- furfurylthiol;
- geraniol;
- geranyl acetate;
- geranyl isobutyrate;
- guaiacol;
- heliotropin;
- heptalactone-gamma;
- heptanone-2;
- heptenal-Z-4;
- hexalactone-gamma;
- hexanal
- hexanol;
- hexenol-E-2;
- hexenol-Z-3;
- hexenyl acetate-E-2;
- hexenyl acetate-Z-3;
- hexenyl capronate-Z-3;
- hexyl acetate;
- hexyl cinnamaldehyde-alpha;
- hotrienol;
- indole;
- ionon-beta;
- ionone-alpha;
- isoamyl acetate;
- isoamyl butyrate;
- isoamyl isovaerianate;
- isobutyl acetate;
- isobutyl thiazole;
- isobutylmethoxypyrazine
- isobutyraldehyde;
- isoeugenol;
- Isomenthon;
- isopropylmethoxypyrazine;
- isopropylmethylpyrazine-2;4;
- isopulegol;
- isova-leraldehyde;
- jasmine lactone;
- jasmon cis;
- ketoisophorone;
- limonene-d;
- linalool oxide;
- linalool-d;
- linalool-l;
- linalyl acetate;
- l-menthol;
- l-menthone;
- l-menthyl acetate;
- maltol;
- massoilactone;
- melonal;
- menthadienyl acetate;
- menthofurolactone;
- menththiol-1;8;
- methional;
- methoxymethylpyrazine-2;3;
- methyl anthranilate;
- methyl butyl acetate-2;
- methyl butyrate;
- methyl cinnamate;
- methyl dihydrojasmonate;
- methyl furanthiol-2;3;
- methyl jasmonate;
- methyl salicylate;
- methyl tetrahydrofuranthiol-2;3;
- methyl thiobutyrate;
- methyl thiohexyl acetate-1;3;
- methylcyclopen-tenolone;
- methylthiohexanol-1;3;
- myrtenal;
- naringin;
- neo-menthol;
- neral;
- nerol;
- nerylacetate;
- nonadienal-2;4;
- nonadienal-2;6;
- nonalactone delta;
- nonalactone gamma;
- nonenal-E-2;
- nonenal-Z-6;
- nonenol-Z-6;
- nootkatone;
- octalactone gamma;
- octalactone delta;
- octanal
- octenol-1;3;
- pellitorin;
- pentenone-1;3;
- pentyl acetate;
- phenyl ethyl acetate;
- phenyl ethyl alcohol;
- phenyl acetaldehyde;
- pinene-alpha;
- pinene-beta;
- piperitanate;
- piperitone
- prenyl thioacetate;
- prenyl thiol;
- reubenamine;
- rose oxide;
- rubescenamine;
- sabinene hydrate;
- scatole;
- sotolone;
- styrolyl acetate;
- terpinenol-4;
- terpineol;
- thiohexanol-1;3;
- thiohexyl acetate-1;3;
- thiomenthanone-8;3;
- thiopentanone-4;4;2;
- thymol;
- tridecatrienal;
- trimethyl pyrazine;
- undecadienal-2;4;
- undecalactone-gamma and delta;
- undecatriene;
- vanillin;
- vinyl guaiacol;
- whisky lactone;
and mixtures thereof.

In particular preferred aroma or flavoring compounds encompass menthol, cineol, eugenol, thymol, cinnamic aldehyde, peppermint oil, spearmint oil, eucalyptus oil, thyme oil, cinnamon oil, clove oil, spruce needle oil, fennel oil, sage oil, aniseed oil, star anise oil, chamomile oil, and caraway oil, and their mixtures.

### Sweeteners

The term "sweeteners" here denotes substances having a relative sweetening power of at least 25, based on the sweetening power of sucrose (which accordingly has a sweetening power of 1). Sweeteners to be used in an orally consumable product (in particular foodstuff, feed or medicament) according to the invention (a) are preferably non-cariogenic and/or have an energy content of not more than 5 kcal per gram of the orally consumable product.

Advantageous sweeteners in a preferred orally consumable product (in particular foodstuff, feed or medicament) according to the invention are selected from the following groups
(a) Naturally occurring sweeteners, preferably selected from the group comprising miracu-lin, monellin, mabinlin, thaumatin, curculin, brazzein, pentaidin, D-phenylalanine, D-tryptophan, and extracts or fractions obtained from natural sources, comprising those amino acids and/or proteins, and the physiologically acceptable salts of those amino acids and/or proteins, in particular the sodium, potassium, calcium or ammonium salts, neohesperidin dihydrochalcone, naringin dihydrochalcone, stevioside, steviolbioside, rebaudiosides, in particular rebaudioside A, rebaudioside B, rebaudioside C, rebaudioside D, rebaudioside E, rebaudioside F, rebaudioside G, rebaudioside H, dulcosides and rubusoside, suavioside A, suavioside B, suavioside G, suavioside H, suavioside I, suavioside J, baiyunoside 1, baiyunoside 2, phlomisoside 1, phlomisoside 2, phlomisoside 3 and phlomisoside 4, abrusoside A, abrusoside B, abrusoside C, abrusoside D, cyclocaryoside A and cyclocaryoside I, osladin, polypodoside A, strogin 1, strogin 2, strogin 4, selligueain A, dihydroquercetin 3-acetate, perillartin, telosmoside A₁₅, perian-drin I-V, pterocaryosides, cyclocaryosides, mukuroziocides, trans-anethole, trans-cinnamaldehyde, bryosides, bryonosides, bryonodulcosides, carnosiflosides, scandeno-sides, gypenosides, trilobatin, phloridzin, dihydroflavanols, hematoxylin, cyanin, chlorogenic acid, albiziasaponin, telosmosides, gaudichaudioside, mogrosides, mogroside V, hernandulcins, monatin, phyllodulcin, glycyrrhetinic acid and derivatives thereof, in particular glycosides thereof such as glycyrrhizine, and the physiologically acceptable salts of those compounds, in particular the sodium, potassium, calcium or ammonium salts; and extracts or concentrated fractions of the extracts, selected from the group comprising thaumatococcus extracts (katamfe plant), extracts from *Stevia* ssp. (in particular *Stevia rebaudiana),* swingle extracts *(Momordica* or *Siratia grosvenorii,* Luo-Han-Guo), extracts from *Glycerrhyzia* ssp. (in particular *Glycerrhyzia glabra),* extracts from *Rubus* ssp. (in particular *Rubus suavissimus),* citrus extracts and extracts from *Lippia dulcis;*
(b) Synthetic sweet-tasting substances, preferably selected from the group comprising magap, sodium cyclamate or other physiologically acceptable salts of cyclamic acid, acesulfame K or other physiologically acceptable salts of acesulfame, neohesperidin dihydrochalcone, naringin dihydrochalcone, saccharin, saccharin sodium salt, aspartame, superaspartame, neotame, alitame, advantame, perillartin, sucralose, lugduname, carrelame, sucrononate and sucrooctate.

### Thickeners

Advantageous thickeners in a preferred orally consumable product (in particular foodstuff, feed or medicament) according to the invention are selected from the group comprising: crosslinked polyacrylic acids and derivatives thereof, polysaccharides and derivatives thereof, such as xanthan gum, agar-agar, alginates or tyloses, cellulose derivatives, for example carboxymethylcellulose or hydroxycarboxymethylcellulose, fatty alcohols, monoglycerides and fatty acids, polyvinyl alcohol and polyvinylpyrrolidone.

Preference is given according to the invention to an orally consumable product (in particular foodstuff or feed) which comprises milk thickened with lactic acid bacteria and/or cream thickened with lactic acid bacteria and which preferably is selected from the group comprising yoghurt, kefir and quark.

A food composition according to the invention comprising milk thickened with lactic acid bacteria and/or cream thickened with lactic acid bacteria is advantageously an orally consumable product which comprises a probiotic, wherein the probiotic is preferably selected from the group comprising Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) and Lactobacillus rhamnosus (ATCC 53013).

### Additives for chewing gums

Particular preference is given to an orally consumable product (in particular foodstuff, feed or medicament) according to the invention that is a chewing gum and comprises a chewing-gum base. The chewing-gum base is preferably selected from the group comprising chewing-gum or bubble-gum bases. The latter are softer, so that gum bubbles can also be formed therewith. Preferred chewing-gum bases according to the invention include, in addition to the natural resins or the natural latex chicle that are traditionally used, elastomers such as polyvinyl acetate (PVA), polyethylene, (low or medium molecular weight) polyisobutene (PIB), polybutadiene, isobutene-isoprene copolymers (butyl rubber), polyvinyethyl ether (PVE), polyvinylbutyl ether, copolymers of vinyl esters and vinyl ethers, styrene-butadiene copolymers (styrene-butadiene rubber, SBR) or vinyl elastomers, for example based on vinyl acetate/vinyl laurate, vinyl acetate/vinyl stearate or ethylene/vinyl acetate, as well as mixtures of the mentioned elastomers, as described, for example, in EP 0 242 325, US 4,518,615, US 5,093,136, US 5,266,336, US 5,601,858 or US 6,986,709. In addition, chewing-gum bases that are preferably to be used according to the invention preferably comprise further constituents such as, for example, (mineral) fillers, plasticisers, emulsifiers, antioxidants, waxes, fats or fatty oils, such as, for example, hardened (hydrogenated) vegetable or animal fats, mono-, di- or tri-glycerides. Suitable (mineral) fillers are, for example, calcium carbonate, titanium dioxide, silicon dioxide, talcum, aluminium oxide, dicalcium phosphate, tricalcium phosphate, magnesium hydroxide and mixtures thereof. Suitable plasticisers, or agents for preventing adhesion (detackifiers), are, for example, lanolin, stearic acid, sodium stearate, ethyl acetate, diacetin (glycerol diacetate), triacetin (glycerol triacetate), triethyl citrate. Suitable waxes are, for example, paraffin waxes, candelilla wax, carnauba wax, microcrystalline waxes and polyethylene waxes. Suitable emulsifiers are, for example, phosphatides such as lecithin, mono- and diglycerides of fatty acids, for example glycerol monostearate.

Chewing gums according to the invention (in particular as disclosed above) preferably comprise constituents such as sugars of different types, sugar substitutes, other sweet-tasting substances, sugar alcohols (in particular sorbitol, xylitol, mannitol), ingredients having a cooling effect, taste correctors for unpleasant taste impressions, further taste-modulating substances (e.g. inositol phosphate, nucleotides such as guanosine monophosphate, adenosine monophosphate or other substances such as sodium glutamate or 2-phenoxypropionic acid), humectants, thickeners, emulsifiers, stabilizers, odor correctors and flavors (e.g. eucalyptus-menthol, cherry, strawberry, grapefruit, vanilla, banana, citrus, peach, blackcurrant, tropical fruits, ginger, coffee, cinnamon, combinations (of the mentioned flavors) with mint flavors as well as spearmint and peppermint on their own). The combination *inter alia* of the flavors with further substances that have cooling, warming and/or mouth-watering properties is of particular interest.

### PHARMACEUTICAL COMPOSITIONS

Pharmaceutical compositions according to the present invention are limited to those which are designed for oral uptake, where unpleasant taste is of importance, particularly in terms of acceptance by the patient. While these compositions can be solid, for example in the form of tablets, pills or powders (including those for rapid dissolution in water) the preferred embodiment, however, represents a liquid formulation such as for example a common cold juice or a hard-shell or soft-shell capsule comprising the active in the liquid core.

The nature of the actives incorporated into such pharmaceutical composition is not critical; its choice simply depends on their particular taste or after-taste which needs modification as suggested by the present invention. Typical examples for these kinds of active include steroidal anti-inflammatory substances of the corticosteroid type, such as e.g. hydrocortisone, hydrocortisone derivatives, such as hydrocortisone 17-butyrate, dexamethasone, dexamethasone phosphate, methylprednisolone or cortisone; non-steroidal anti-inflamma-tories like oxicams, such as piroxicam or tenoxicam; salicylates, such as aspirin, Disalcid, Solprin or fendosal; acetic acid derivatives, such as diclofenac, fenclofenac, indomethacin, sulindac, tolmetin or clindanac; fenamates, such as mefenamic, meclofenamic, flufenamic or niflumic; propionic acid derivatives, such as ibuprofen, naproxen or benoxaprofen, or pyrazoles, such as phenylbutazone, oxyphenylbutazone, febrazone or azapropazone. Alternatively, natural anti-inflammatory substances or reddening- and/or itching-alleviating substances can be employed. Plant extracts, specific highly active plant extract fractions and highly pure active substances isolated from plant extracts, can be employed like extracts, fractions and active substances from aloe vera, Commiphora species, Rubia species, Rubus species, willow, rose-bay, willowherb, oats, calendula, arnica, St. John's wort, honeysuckle, ginger, chamomile, rosemary, sage, melissa, Passiflora incarnata, Sophora japonica, witch hazel, Pueraria, Dian-thus or Echinacea, as well as pure substances, such as, inter alia, bisabolol, apigenin, apigenin-7-glucoside, rosmarinic acid, boswellic acid, phytosterols, glycyrrhizic acid, glabridin, licochalcone A, [6]-paradol, and anthranilic acid amides, such as, in particular, avenan-thramides or dianthramides, are particularly preferred. The total amount of anti-irritants in a formulation or product according to the invention is preferably in the range of from 0.0001 to 20 wt.%, preferably from 0.0001 to 10 wt.%, in particular from 0.001 to 5 wt.%, based on the total weight of the formulation or product, respectively.

Particular useful co-actives are selected from the group consisting of anti-mycotica and pain relief agents, and more particularly the group consisting of erythromycin, dimetindene, betamethasone, ibuprofen, ketoprofene, diclofenac, metronidazole, acyclovir, imiquimod, terbinafine, docosanol, cyclopyroxolamine, and their mixtures:

**Erythromycin** is a macrolide antibiotic that has an antimicrobial spectrum similar to or slightly wider than that of penicillin, and is often used for people who have an allergy to penicillin.

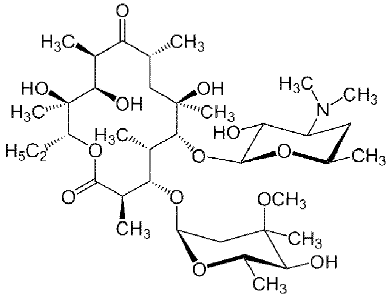

**Dimetindene,** also known as Fenistil (RS-dimethyl(2-(3-[pyridin-2-yl)ethyl]-1H-inden-2-yl)ethyl)amine) is an antihistamine/anticholinergic used orally and locally as an antipruritic.

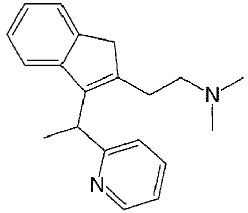

**Betamethasone** (8S,9R, 10S.11S, 13S, 14S, 16S, 17R)-9-fluoro-11,17-(2-hydroxyacetyl)-10, 13,16-trimethyl-6,7,8,9,10,11,12,13,14,15,16,17-dodecahydro-3H-cyclopenta(alpha)-phenanthren-3-one) is a potent glucocorticoid steroid with anti-inflammatory and immunosuppressive properties.

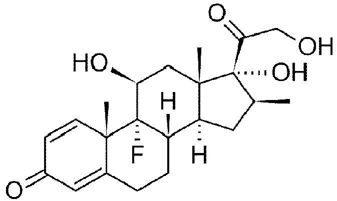

**Ibuprofen** (RS)-2-(4-(2-methylpropyl)phenyl)propanoic acid) from the nomenclature iso-butyl-propanoic-phenolic acid) is a non-steroidal anti-inflammatory drug (NSAID) used for relief of symptoms of arthritis, fever, as an analgesic (pain reliever), especially where there is an inflammatory component, and dysmenorrhea.

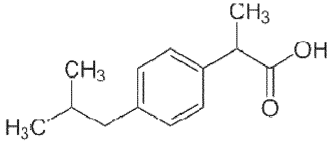

**Ketoprofen** (RS)2-(3-benzoylphenyl)-propionic acid is another one of the propionic acid class of non-steroidal anti-inflammatory drug (NSAID) with analgesic and antipyretic effects.

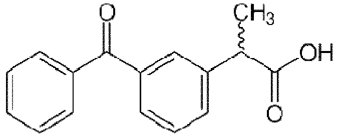

**Diclofenac** is also a non-steroidal anti-inflammatory drug (NSAID) taken to reduce inflammation and as an analgesic reducing pain in certain conditions.

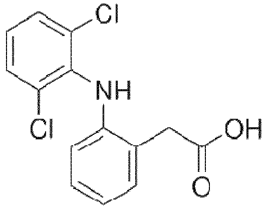

**Metronidazole** (2-(2-methyl-5-nitro-1H-imidazol-1-yl)ethanol) is a nitroimidazole antibiotic medication used particularly for anaerobic bacteria and protozoa.

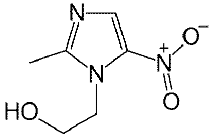

**Acyclovir** or acyclovir (USAN, former BAN), chemical name acycloguanosine (2-Amino-1,9-dihydro-9-((2-hydroxyethoxy)methyl)-6H-Purin-6-one), abbreviated as ACV is a guanosine analogue antiviral drug, marketed under trade names such as *Cyclovir, Herpex, Acivir, Acivirax, Zovirax,* and *Xovir.* The solid active agent has a solubility in water (20° dH) at 20 °C of less than 5 g/L.

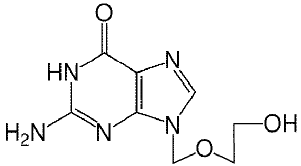

**Imiquimod** (3-(2-methylpropyl)-3,5,8-triazatricyclo[7.4.0.0.^{2,6}]trideca-1(9),2(6),4,7,10, 12- hexaen-7-amine, INN) is a prescription medication that acts as an immune response modifier.

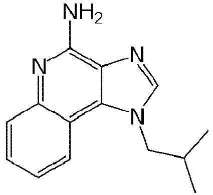

**Terbinafine,** more particularly terbinafine hydrochloride [(2E)-6,6-dimethylhept-2-en-4-yn-1-yl](methyl)(naphthalen-1-ylmethyl)amine) is a synthetic allylamine antifungal from Novartis. It is highly lipophilic in nature and tends to accumulate in skin, nails, and fatty tissues.

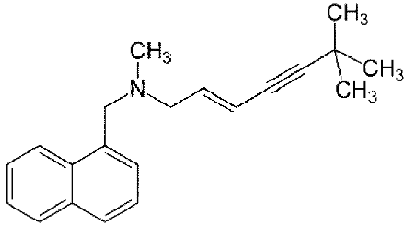

**Docosanol,** also known as behenyl alcohol, is a saturated fatty alcohol used traditionally as an emollient, emulsifier, and thickener in cosmetics, nutritional supplement (as an individual entity and also as a constituent of policosanol), and more recently, in a Food and Drug Administration (FDA) approved pharmaceutical, Abreva, approved as an antiviral agent for reducing the duration of cold sores caused by the herpes simplex virus.

**Ciclopiroxolamine** (6-cyclohexyl-1-hydroxy-4-methylpyridin-2(1H)-one) also called Batrafen, Loprox, Mycoster, Penlac and Stieprox, is a synthetic antifungal agent for topical dermatologic treatment of superficial mycoses.

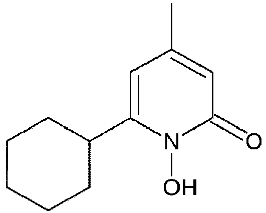

### INDUSTRIAL APPLICATION

Another object of the present invention refers to a method for masking unpleasant, particularly bitter and/or astringent and/or liquorice-like taste impressions of inorganic salts and/or mineral substances and food products or pharmaceutical compositions containing them, comprising or consisting of the following steps:
(i) providing a food product or a pharmaceutical composition containing an inorganic salt and/or a mineral substance with said unpleasant, particularly bitter and/or astringent and/or liquorice-like taste impressions,
(ii) providing oleanol glycosides or their salts or an extract comprising said oleanol glycosides according to formula 1; and
(iii) adding about 0.01 to about 500 ppm of the glycosides of step (ii) to the product or composition of step (i).

Another object of the present invention refers to the use of oleanol glycosides and/or their salts and/or an extract containing said oleanol glycosides according formula 1 for masking unpleasant, particularly bitter and/or astringent and/or liquorice-like taste impressions of inorganic salts and of food products or pharmaceutical compositions comprising them.

The oleanol glycosides, their salts, or the extract containing the oleanol glycosides is added to the products or compositions such that the concentration of oleanol glycosides therein is from about 0.01 to about 500 ppm, preferably, from about 0.05 ppm to about 100 ppm, particularly preferably from about 0.1 to about 50 ppm.

For the sake of good order, it is emphasized that any preferred embodiment, particularly with respect to combinations, mixtures, ratios, ranges, conditions and the like cited above also count with regard with the proposed method and use. Thus, repetition is not necessary.

### EXAMPLES

### Example 1

### Isolation of oleanol glycosides from escin mixture

Escin is a commercially available mixture of oleanol glycosides prepared by extraction of seeds of *Aesculus hippocastanum.* For the isolation of pure oleanol glycosides β-escin was bought from Sigma.

### Preparative HPLC

| | |
|---|---|
| Separation ID: | H-3070-B, -C, and -D |
| Sample: | 2g of escin mixture (Sigma) |
| Stationary phase: | Kromasil C18, 10 µm, 250 x 50 mm |
| Mobile phase A: | 5mMol ammonium formate buffer, adjusted with formic acid to pH 3.0 |
| Mobile phase B: | acetonitrile with 5 mMol ammonium formate |
| Gradient: | 39% to 49% B in 57 min |
| Flowrate: | 80 ml/min |
| Detection: | evaporative light scattering detector (ELSD) |

Collected fractions were analyzed by HPLC-MS. Fractions containing Oleanol glycosides were combined and the solvent was evaporated in vacuum and analyzed by H-NMR spectroscopy. The following compounds were isolated from escin mixture:

| No. | Structure | Name / CAS | yield [mg] |
|---|---|---|---|
| NP-004601 | 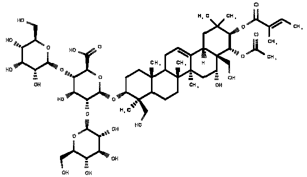 | Escin Ia | 170 mg |
| NP-014327 | 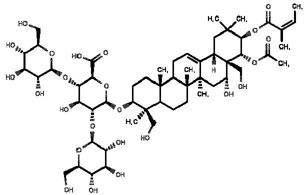 | Escin Ib | 109 mg |
| NP-018919 | 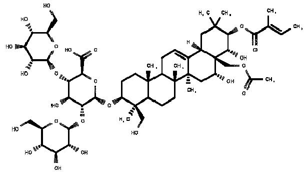 | Escin IVa, Isoescin la | 103 mg |
| NP-004600 | 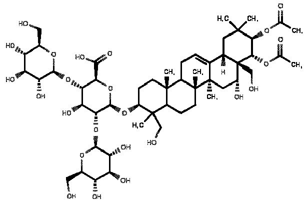 | Escin IV | 45 mg |
| | | 219944-22-6 | |
| NP-018918 | 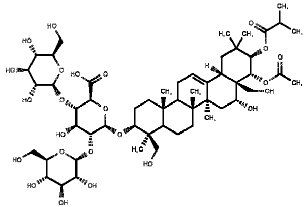 | Escin V | 26 mg |
| | | [219944-26-0] | |

All isolated compounds were characterized by the LCMS method listed in table 1.

**Table 1**

| LCMS method for analytical characterization of pure compounds | | | |
|---|---|---|---|
| **HPLC system** | PE Series 200 | | |
| **MS system** | Applied Biosystems API 150 | | |
| **Data system** | Analyst 1.3 | | |
| **Stationary phase** | Merck Select B 250x4 mm, 5 µm | | |
| **Flux rate** | 1 ml/min | | |
| **Detection** | (+/(-)-ESI, Fast-Switching-Mode | | |
| | ELSD (Sedex 75) | | |
| | UV (Merck, 254 nm) | | |
| **Sample concentration** | 10 mg/ml in DMSO | | |
| **Injection volume** | 30 µl | | |
| **Mobile phase:** | A: 5 mM ammonium formate and 0.1 % formic acid | | |
| | B: acetonitrile/methanol = 1:1, 5 mM ammonium formate und 0.1 % formic acid (pH 3) | | |
| **Gradient** | Time [min] | % A | % B |
| | 00.0 | 85 | 15 |
| | 30.0 | 0 | 100 |
| | 35.0 | 0 | 100 |

### Example 2

### Taste of the combination of escin with sodium chloride

The sensory evaluation of the samples was performed by a team of four experienced assessors. For tasting the compound in a 0.25% Sodium chloride solution was compared against a 0.25 % sodium chloride solution. Descriptors (saliva formation, lingering, salty, seawater, feeling thirsty, coating, warm/structure/mouthfeel, metallic) where evaluated based on a 0.35% sodium chloride solution as benchmark. The results are provided in **Table 2:**

**Table 2**

| Tasting of oleanol glycosides + sodium chloride (NaCl) | | |
|---|---|---|
| **Test method** | Descriptive and discriminative evaluation, "sip and spit" method, blinded and randomized samples, pH neutral, | |
| | Confidence scoring: 75% or higher | |
| | Recorded: sensory descriptions of assessors | |
| **Test sample** | 0.25% sodium Chloride solution | |
| | 0.25% sodium chloride solution + escin | |
| | 0.35% sodium chloride solution as benchmark | |
| **Panelists** | 4 experienced assessors, trained to recognize the off-flavor | |
| **Preparation of the samples** | Two different concentrations of escin (1ppm, 10 ppm) with 0.25% sodium chloride dissolved in spring water | |
| **Evaluation** | Descriptors were evaluated. All results shown are based at a score of 75% or more. | |
| | 1ppm | Seawater taste is enhanced |
| | 10 ppm | coating and metallic taste are improved; vegetable aftertaste |

### Example 3

### Taste of the combination of oleanol glycosides with sodium and potassium chloride

The sensory evaluation of the samples was performed by a team of seven experienced assessors. The results are provided in **Table 3:**

**Table 3**

| Tasting of high-purity oleanol glycosides + sodium chloride + potassium chloride (KCl) | | |
|---|---|---|
| **Test method** | Descriptive and discriminative evaluation, "sip and spit" method, blinded and randomized samples, pH neutral, | |
| | Confidence scoring: 75% or higher | |
| | Recorded: sensory descriptions of assessors | |
| **Test sample** | 0.1% KCl + 0.25% NaCl + escin | |
| | 0.1% KCl + 0.25% NaCl as control | |
| **Panelists** | 7 experienced assessors, trained to recognize the off-flavor, in particular the bitterness, of potassium chloride | |
| **Preparation of the samples** | 10 ppm of escin with 0.1% potassium chloride + 0.25% sodium chloride dissolved in spring water | |
| **Evaluation** | 10ppm | improves the seawater taste, coating of the tongue and the metallic aftertaste of the salt mix |

### Example 4

### Taste modulation of crackers

The sensory evaluation of the samples was performed by 4 experienced assessors. The results are provided in **Table 4:**

**Table 4**

| Taste evaluation of cracker salted with sodium chloride | |
|---|---|
| **Test method** | Descriptive evaluation (saliva formation, feeling, thirsty, coating, warm/structure/mouthfeel, metallic, bitter, tingling, musty, dry, astringent, vegetable, carton, pungent) |
| **Test sample** | Cracker with 0.7% NaCl + escin |
| | Cracker with 0.7% NaCl as negative control |
| | Cracker with 1% NaCl as positive control |
| **Panelists** | 15 naïve as well as experienced |
| **Preparation of the samples** | Cracker |
| | 150g flower type 405 |
| | 35g neutral plant oil |
| | 25ml water |
| | 0.7-1% sodium chloride |
| | +/- escin (10-20 ppm) |
| **Evaluation** | Cracker with escin was preferred, due to its positive influence on saliva formation. |

The sensory profile of the cracker with sodium chloride was strongly improved by the product. The product would enable a strong reduction of sodium chloride content in crackers and related categories.

### Example 5

### Taste modulation of vegetable bouillon

The sensory evaluation of the samples was performed by 5 experienced assessors. The results are provided in **Table 5:**

**Table 5**

| Taste evaluation of vegetable broth salted with sodium chloride | |
|---|---|
| **Test method** | Descriptive evaluation (salvia formation, lingering, salty, seawater, feeling thirsty, coating, warm/structure/mouthfeel, metallic, bitter aftertaste, tingling, musty, dryness, astringent, vegetable, carton, pungent) |
| **Test sample** | Escin (25 ppm) |
| **Panelists** | 5 experienced assessors |
| **Preparation of the samples** | (a) Broth with a dosage of 10.5 g/l and a salt content of 0.5% sodium chloride |
| | (b) Is (a) + escin |
| | (c) Is (a) with additional 0.2% sodium chloride |
| **Evaluation** | The descriptors salty, lingering, coating, warm/structure/ mouthfeel are positive. The compound give some bitter, dry and carton aftertaste. Overall conclusion is positive |

The sensory profile of the soup salted with sodium chloride was strongly improved by the product. This was also evident in soups containing a small amount of sodium chloride. Thus, the product would enable a strong reduction of sodium chloride content in soups and related categories.

## Claims

1. A preparation, comprising or consisting of
(a) at least one oleanol glycoside and/or its salt and/or an extract containing at least one oleanol glycoside and
(b) at least one inorganic salt or mineral substance,
wherein compound (a) follows formula **1**. 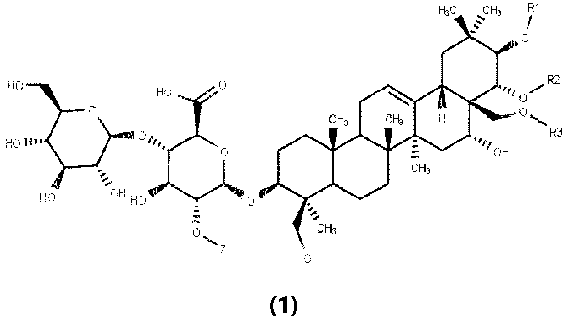
wherein Z represents at least one monosaccharide selected from Glc, Gal, Xyl, Ara and Rha; R1 has the meaning of Ac, Ang, Tig, MeBu, and/or iBu, R2 and R3 independently stand for H or Ac,
on condition that a compound (a) according to formula (I) in which Z means Glc, R1 means Tig, R2 is H, and R3 is H is disclaimed.

2. The preparation of Claim 1, wherein compound (a) is selected from the following list:
| Structure | Name / CAS |
|---|---|
| 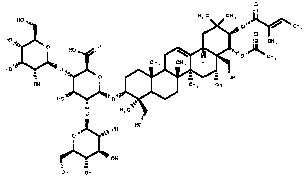 | Escin Ia |
| 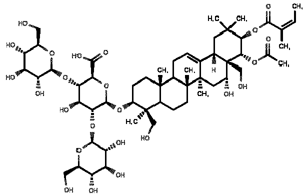 | Escin Ib |
| 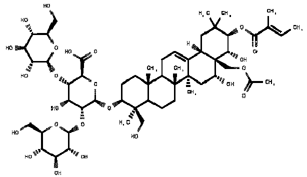 | Escin IVa, Isoescin Ia |
| 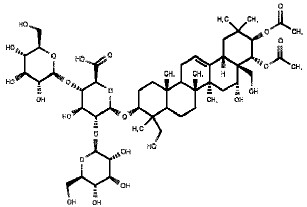 | Escin IV |
| | 219944-22-6 |
| 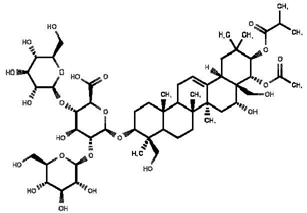 | Escin V |
| | [219944-26-0] |

3. The preparation of Claim 1 or 2, wherein the salts of said oleanol glycosides are selected from the group comprising the alkali metals salts, alkaline earth metals salts or ammonium salts.

4. The preparation of any of Claims 1 to 3, wherein the cations of said inorganic salts or mineral substances forming group (b) are selected from the group consisting of sodium, potassium, magnesium, and calcium and the anions are selected from the group consisting of chloride, sulfate, phosphate, carbonate, and glutamate.

5. The preparation of any of Claims 1 to 4, wherein the inorganic salts or mineral substances represent sodium chloride, potassium chloride or their mixtures.

6. The preparation of any of Claims 1 to 5, wherein said preparation comprises component (a) in concentration of from about 0.01 to about 500 ppm.

7. The preparation of any of Claims 1 to 6, wherein said preparation comprises component (b) in concentration of from about 0.01 wt.-percent to about 5 wt.-percent.

8. The preparation of any of Claims 1 to 7, wherein said preparation comprises component (a) in concentration of from about 0.01 to about 500 ppm and component (b) in concentration of from about 0.01 wt.-percent to about 5 wt.-percent.

9. A food product comprising the preparation of any of Claims 1 to 8.

10. The food product of Claim 9 representing a bakery product, a convenience product or a soup.

11. A pharmaceutical composition comprising the preparation of any of Claims 1 to 8.

12. The pharmaceutical composition of Claim 11 representing a liquid formulation.

13. The food product of Claim 9 or the pharmaceutical composition of Claim 11, comprising said oleanol glycosides or their salts in concentrations of from about 0.01 to about 500 ppm.

14. A method for masking unpleasant, particularly bitter and/or astringent and/or liquorice-like taste impressions of inorganic salts and/or mineral substances and food products or pharmaceutical compositions containing them, comprising or consisting of the following steps:
(i) providing a food product or a pharmaceutical composition containing an inorganic salt and/or a mineral substance with said unpleasant, particularly bitter and/or astringent and/or liquorice-like taste impressions,
(ii) providing oleanol glycosides or their salts or an extract comprising said oleanol glycosides according to Claim 1 or 2; and
(iii) adding about 0.01 to about 500 ppm of the glycosides of step (ii) to the product or composition of step (i).

15. Use of oleanol glycosides and/or their salts and/or an extract containing said oleanol glycosides according to Claim 1 or 2 for masking unpleasant, particularly bitter and/or astringent and/or liquorice-like taste impressions of inorganic salts and of food products or pharmaceutical compositions comprising them.
